# EUROPEAN PATENT APPLICATION

(11) **EP 4 840 504 A1**
(43) Date of publication of application: **18.11.2026**
(21) Application number: 26178196.7
(22) Date of filing: 12.05.2026
(51) Int. Cl.: A61N 5/10

(54) **TARGET VOLUME POSITIONING METHOD, TARGET VOLUME POSITIONING SYSTEM, AND ELECTRONIC DEVICE**

(30) Priority: 16.05.2025 CN 202510638682
(71) Applicant: Our United Corporation, Xi'an, Shaanxi 710018 (CN); OUR United (Changchun) GAMADAO Co., Ltd, Changchun City (CN)
(72) Inventor: LIU, Haifeng, Shaanxi, 710018 (CN); CHEN, Xianzhi, Shaanxi, 710018 (CN); LI, Hanwen, Shaanxi, 710018 (CN); XU, Zhifu, Shaanxi, 710018 (CN); LIU, En, Shaanxi, 710018 (CN); WEI, Yongpeng, Shaanxi, 710018 (CN)
(74) Representative: Murgitroyd & Company

(57) **Abstract**

A target volume positioning method, a target volume positioning system, and an electronic device are provided in the present disclosure. The method includes: acquiring current image data of a target object before irradiation is to be performed on a target volume of the target object; inputting the current image data into a first target volume positioning model, where the first target volume positioning model is configured to determine a position offset of the target volume based on a dynamic mapping relationship for the target volume; the dynamic mapping relationship for the target volume is: a correspondence between a position of the target volume of the target object and the image data with motion changes of the target volume.

## Description

### TECHNICAL FIELD

The present disclosure relates to the technical field of medical treatment, and in particular, to a target volume positioning method, a target volume positioning system, and an electronic device.

### BACKGROUND

With the rapid development of precision medicine, it is necessary to accurately position a target volume of a tumor before performing radiation therapy for the tumor, so that precise radiation therapy may be performed on the target volume.

However, in current clinical practice, a position of a tumor target volume is mainly determined through a low-dose KV image.

### SUMMARY

In a first aspect, a target volume positioning method is provided, which includes: acquiring current image data of a target object before irradiation is to be performed on a target volume of the target object; inputting the current image data into a first target volume positioning model, where the first target volume positioning model is configured to determine a position offset of the target volume based on a dynamic mapping relationship for the target volume; the dynamic mapping relationship for the target volume is: a correspondence between a position of the target volume of the target object and the image data with motion changes of the target volume.

In a second aspect, an electronic device is further provided in the present disclosure, and the electronic device includes: a processor and a memory configured to store instructions executable for the processor; where the processor is configured to execute the instructions to implement the target volume positioning method in the above-mentioned first aspect.

In a third aspect, a non-transitory storage medium is further provided in the present disclosure, and the storage medium stores computer programs; where when the computer programs are read and executed, the target volume positioning method in the above-mentioned first aspect is implemented.

### BRIEF DESCRIPTION OF THE DRAWINGS

The accompanying drawings are intended for a better understanding of the present scheme, and do not constitute the limitation of the present disclosure, where:
FIG. 1 is a schematic diagram of a scenario of a target volume positioning system provided in the embodiments of the present disclosure;
FIG. 2 is a block diagram of a radiation therapy device provided in the embodiments of the present disclosure;
FIG. 3 is a block diagram of another target volume positioning system provided in the embodiments of the present disclosure;
FIG. 4 is a block diagram of yet another target volume positioning system provided in the embodiments of the present disclosure;
FIG. 5 is a block diagram of yet another target volume positioning system provided in the embodiments of the present disclosure;
FIG. 6 is a flowchart of a target volume positioning method provided in the embodiments of the present disclosure;
FIG. 7 is a flowchart of another target volume positioning method provided in the embodiments of the present disclosure;
FIG. 8 is a flowchart of yet another target volume positioning method provided in the embodiments of the present disclosure;
FIG. 9 is a flowchart of yet another target volume positioning method provided in the embodiments of the present disclosure;
FIG. 10 is a block diagram of a second target volume positioning model provided in the embodiments of the present disclosure;
FIG. 11 is a block diagram of a target volume positioning apparatus provided in the embodiments of the present disclosure; and
FIG. 12 is a block diagram of an electronic device provided in the embodiments of the present disclosure.

### DETAILED DESCRIPTION

The technical solutions in the embodiments of the present disclosure will be described below clearly and completely in conjunction with the accompanying drawings. Obviously, the described embodiments are merely a part of the embodiments of the present disclosure, but not all of the embodiments of the present disclosure. Based on the embodiments of the present disclosure, all other embodiments obtained by those skilled in the art without creative labor are within the protection scope of the present disclosure.

In the description of the present disclosure, it will be understood that, orientations or positional relationships indicated by the terms such as "center", "longitudinal", "transverse", "width", "up", "down", "front", "behind", "left", "right", "vertical", "horizontal", "top", "inner", and "outer" are based on orientations or positional relationships shown in the accompanying drawings, which are merely for convenience in description of the present disclosure and simplifying the description, but not to indicate or imply that the indicated apparatuses or elements must have a specific orientation, or be constructed and operated in a specific orientation, thus cannot be understood as limitation of the present disclosure. In addition, terms "first", "second" and "third" are merely used for a purpose of description, and cannot be understood as indicating or implying the relative importance or implicitly indicating the number of referred technical features. Thus, features defined with "first", "second", or "third" may explicitly or implicitly include one or more of the features. In the description of the present disclosure, "a plurality of" means two or more objects unless otherwise specified.

In the description of the present disclosure, the word "exemplary" is used to represent "being as an example, instance, or illustration." Any embodiment in the present disclosure described as "exemplary" is not necessarily to be illustrated as preferred or advantageous over other embodiments. The following description is presented to enable any skilled in the art to implement and use the present disclosure. In the following description, details are set forth for purposes of explanation. It should be understood that the ordinary technical personnel in the art may recognize that the present disclosure may be implemented without using these specific details. In other instances, well-known structures and processes are not described in detail to avoid obscuring the description of the present disclosure with unnecessary details. Thus, the present disclosure is not intended to be limited to the shown embodiments, but to be consistent with the widest scope of principles and features disclosed in the present disclosure.

Terms involved in the embodiments of the present disclosure may be explained below to facilitate readers' understanding.

### 1. Target volume

Herein, a target volume (i.e., an anatomical target volume) refers to a tumor area that requires to be treated by radiation therapy, thus to accurately kill tumor cells while protecting surrounding normal tissues as much as possible. Usually, the target volume may include a tumor target volume, a clinical target volume, an internal target volume, a planning target volume, etc.

### 2. Heterogeneous Region

Heterogeneous regions refer to regions within or around a tumor that exhibit significant differences in biological features (such as metabolic activity, hypoxia, gene expression, cell proliferation) or microenvironment (such as vascular distribution, immune infiltration). For example, heterogeneous regions may include high-risk sub regions such as hypoxic regions, proliferative regions, and edge invasion regions.

### 3. Tumor biological target volume

Normally, tumors exhibit high biological heterogeneity, with significant differences in cell proliferation rate, blood supply, metabolic activity, and radiation sensitivity among different regions within the tumor. Traditional anatomical imaging may only provide contour information of the target volume and cannot reveal the biological features inside the tumor.

The biological target volume (BTV) of a tumor is a heterogeneous region that dynamically defines a target volume for radiation therapy based on reflecting the biological features of the tumor, such as metabolic activity, hypoxic state, proliferation ability, etc. The tumor biological target volume may be considered as an "internal subdivision" of a tumor target volume, which is a target volume in the (traditional) target volume, more accurately reflecting the heterogeneity and dynamic changes of the tumor, and promoting the transformation of radiation therapy from the traditional "anatomical target volume" to the "biological target volume" paradigm.

For example, tumor biological target volume may include a hypoxic target volume, a proliferative target volume, an edge invasive target volume, or the like. Based on different types of tumor biological target volumes, a corresponding radiation dose in a formulation of an irradiation plan is determined. In this way, targeted radiation therapy may be applied to a tumor.

### 4. Introduction to the entire process of radiation therapy

Radiotherapy (radiation therapy) is one of the important means of tumor treatment, and its core process may be divided into multiple stages, with the aim of ensuring that radiation accurately acts on tumor tissue while protecting surrounding normal tissue to the greatest extent. The following are the key stages of radiation therapy:
1. Diagnostic stage: to determine the necessity, indications, and treatment goals (curative or palliative) of radiation therapy.
2. Positioning stage (simulation): to obtain a positioning image of a patient in a treatment position and to establish a reference coordinate system for radiation therapy.
3. Plan design and verification stage (Treatment Planning): to develop an irradiation (also known as radiotherapy or radiation therapy) plan based on the positioning image, and to verify the feasibility and accuracy of the irradiation plan.
4. Radiation therapy stage: specifically, it includes a setup stage (patient setup) and a radiation therapy stage (beam delivery). During a setup stage, the patient is fixed and positioned to ensure that a body position of the patient is completely consistent with the setup stage, reducing setup errors; during the radiation therapy stage, the patient is precisely irradiated according to the irradiation plan to ensure that a dose distribution is consistent with the irradiation plan.

Usually, the target volume may be positioned through medical imaging data for irradiation. However, due to the dynamic physiological activities of patients (such as coughing, breathing, gastrointestinal peristalsis, involuntary positional changes, heart beats, etc.), the position of the target volume may shift, thereby affecting the effectiveness of radiation therapy.

In view of this, a target volume positioning method is provided in the embodiments of the present disclosure. Regarding this method, the acquired current image data of the target object in real time is input into the target volume positioning model to quickly determine the position offset of the target volume based on the dynamic mapping relationship for indicating the correspondence between the position of the target volume and the image data. In this way, based on the position offset of the target volume, it is possible to accurately determine whether the target volume at the current position may be irradiated, thereby ensuring the accuracy of radiation therapy.

The implementions of the embodiments of the present disclosure will be described in detail below with reference to the accompanying drawings in this specification.

It should be pointed out that, the embodiments of the present disclosure may learn from or refer to each other, for example, same or similar steps, method embodiments, system embodiments and device embodiments may refer to each other, which may not be limited herein.

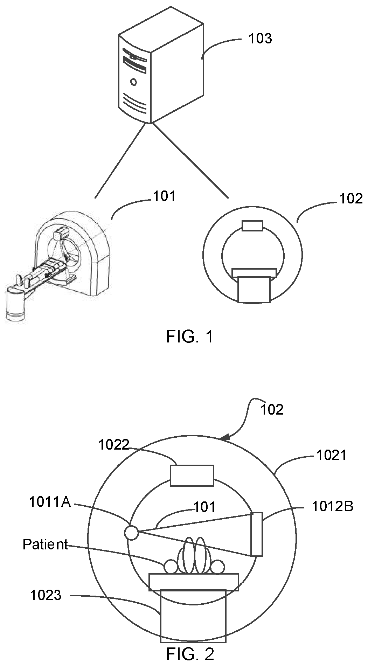

FIG. 1 is a schematic diagram of a target volume positioning system provided in the embodiments of the present disclosure, and the target volume positioning system may include: an image acquisition device 101, a radiation therapy device 102, and a proximal device 103. Herein, the proximal device 103 is connected to the image acquisition device 101 and the radiation therapy device 102. The image acquisition device 101 and the radiation therapy device 102 are both arranged in the same radiation therapy space, such as the same treatment room.

In the embodiments of the present disclosure, the radiation therapy device 102 may be configured to perform irradiation on a target volume.

In some examples, as shown in FIG. 2, the radiation therapy device 102 may include a bracket 1021, a treatment head 1022, and a supporting apparatus 1023.

Herein, the bracket 1021 may be a rotatable bracket. The treatment head 1022 may be arranged on the bracket, which configured to emit an irradiation beam for treatment and perform irradiation on a target object, the irradiation beam may be gamma rays, MV level X-rays, proton rays, etc. For example, the treatment head 1022 may be any one or more of a gamma knife treatment head used for rotational focused radiation therapy, an accelerator treatment head used for adaptive intensity-modulated radiation therapy, or other radiation therapy heads. In the embodiments of the present disclosure, the treatment head 1022 may correspond to different radiation therapy modes, such as rotational focused radiation therapy and adaptive intensity-modulated radiation therapy. The supporting apparatus 1023 is configured to fix, support, and move the target object, which may be a treatment bed.

In some embodiments, when the target object is on the supporting apparatus 1023, the rotation of the bracket 1021 may drive the treatment head 1022 to perform irradiation in 360 degrees around the target object, thereby completing radiation therapy.

In the embodiments of the present disclosure, the image acquisition device 101 may be configured to acquire image data of a target object (such as a patient, a simulated patient model, etc.). The image acquisition device 101 may be configured for real-time image guidance in setup for the target object and/or radiation therapy.

In some embodiments, the image acquisition device 101 may be a body surface optical device, a cone beam computed tomography (CBCT) device, or the like.

In some examples, the image acquisition device 101 may be integrated into the radiation therapy device 102. As shown in FIG. 2, in a case where the image acquisition device 101 is a CBCT device, the CBCT device is arranged on the bracket 1021, and includes a tube 1011A and a detector 1012B. The detector 1012B may receive a imaging beam emitted by the tube 1011A passing through the target object and generate a KV level X-ray projection image, the X-ray projection image is used to indicate a shape and state and/or a position of the target volume of the target object.

In other examples, the image acquisition device 101 may not be integrated into the radiation therapy device 102, but may be arranged in the same space as the radiation therapy device 102. In a case where the image acquisition device 101 is a body surface optical device, the number of body surface optical devices may be one or more, configured to acquire current body surface optical data of the target object in a setup scene before treatment and/or in an irradiation scene during radiation therapy.

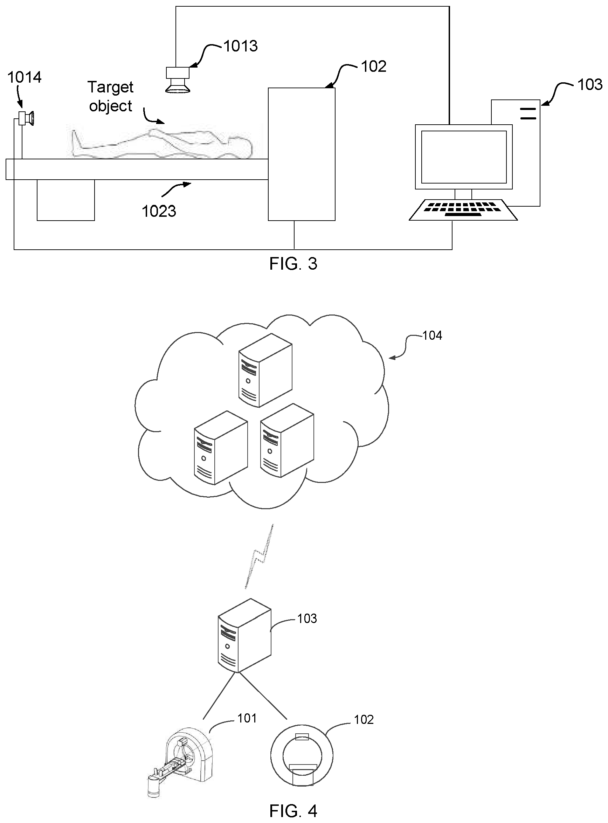

As shown in FIG. 3, the body surface optical device includes a first body surface optical acquisition device 1013 and a second body surface optical acquisition device 1014. Herein, the first body surface optical acquisition device 1013 may be installed above the supporting apparatus 1023 of the radiation therapy device 102, such as on the ceiling of the space where the radiation therapy device 102 is located. The first body surface optical acquisition device 1013 may acquire the current body surface optical data of the target object in the setup scene before radiation therapy. The second body surface optical acquisition device 1014 may be placed on the treatment bed of the radiation therapy device 102 facing the target object, and may move with the supporting apparatus 1023, for example, by using a bracket to be placed at the foot of the treatment bed of the radiation therapy device 102. The second body surface optical acquisition device 1014 may acquire the current body surface data of the target object in the irradiation scene of radiation therapy. Of course, the body surface optical device may further be set in other positions to ensure timely acquisition of the current body surface data of the target object during positioning.

In some examples, the target volume positioning system provided in the embodiments of the present disclosure may further include a radiation therapy guidance device, and the radiation therapy guidance device may be configured to capture a radiation therapy guidance image of the target object. The radiation therapy guidance device may be connected to the proximal device 103.

In the embodiments of the present disclosure, the radiation therapy guidance device may be the same device as the image acquisition device 101 mentioned above, or it may be a different device. For example, the radiation therapy guidance device may include any one or more of a CT device, a magnetic resonance imaging (MRI) device, a PET device, an infrared acquisition device, etc. Correspondingly, a radiation therapy guidance image may include a CT image, an MRI image, a PET image, an infrared image, etc.

In the embodiments of the present disclosure, the proximal device 103 may be configured with a first target volume positioning model. The proximal device may be arranged in the same radiation therapy space as the image acquisition device 101 and the radiation therapy device 102, such as the treatment room, or in a control room outside the treatment room.

In some embodiments, the proximal device 103 may acquire the current image data of the target object from the image acquisition device 101 before the irradiation is about to be performed on the target volume of the target object, and input the current image data into the first target volume positioning model to determine the position offset of the target volume.

Furthermore, in combination with the above-mentioned radiation therapy guidance device, during the process of acquiring the current image data of the target object, the radiation therapy guidance device may send the collected radiation therapy guidance image to the proximal device 103. After acquiring the radiation therapy guidance image of the target object, the proximal device 103 may input the current image data and the radiation therapy guidance image into the first target volume positioning model to determine the position offset of the target volume.

In some embodiments, the proximal device 103 may determine a state of the target object based on the current image data of the target object, and input the current image data into the first target volume positioning model in response to that the state of the target object meets a preset state.

Herein, the state of the target object includes an abnormal motion state and/or a breath holding state.

In some embodiments, the first target volume positioning model may be configured to determine the position offset of the target volume according to the dynamic mapping relationship for the target volume. The dynamic mapping relationship for the target volume may be: a correspondence between a position of the target volume of the target object and the image data with motion changes of the target volume.

In some embodiments, the first target volume positioning model may further be configured to output indication information on whether to perform irradiation on the target volume based on the position offset of the target volume.

For example, in a case where the position offset of the target volume is within a range of a preset threshold, the first target volume positioning model may output first indication information for indicating to perform the irradiation on the target volume.

For example, in a case where the position offset of the target volume is not within the range of the preset threshold, the first target volume positioning model may output second indication information for indicating to adjust the position of the target volume.

Furthermore, in order to ensure accurate irradiation for the target volume, the proximal device 103 may control the radiation therapy device 102 to perform irradiation on the target volume in a case where a time interval between an acquisition time for the current image data of the target volume and an outputting time for the first indication information is shorter than a preset time interval.

In some embodiments, the proximal device 103 may control the radiation therapy device 102 to perform irradiation on the target volume in a case where the target object is in a breath holding state without an abnormal motion state.

In some examples, the proximal device 103 is connected in communication with the image acquisition device 101 and the radiation therapy device 102. Before the radiation therapy device 102 performs irradiation on the target volume of the target object, the image acquisition device 101 acquires the current image data of the target object and sends it to the proximal device 103, so that the proximal device 103 performs the target volume positioning method mentioned later.

In some embodiments, the proximal device 103 may be a computer device with a graphical user interface (GUI), and the computer device includes: one or more processors, a memory, and one or more application programs. For example, the proximal device 103 may include a target volume positioning system application, and a processor of the proximal device 103 executes the target volume positioning system application to implement the target volume positioning method provided in the embodiments of the present disclosure.

In the embodiments of the present disclosure, an entity of the proximal device 103 may be a terminal or a server with a display, which is not limited in the embodiments of the present disclosure.

In some embodiments, the above-mentioned terminal may be at least one of devices such as a smartphone, a smart watch, a desktop computer, a laptop, a virtual reality terminal, an augmented reality terminal, a wireless terminal, or a portable laptop.

In some embodiments, the above-mentioned server may be an independent physical server, or a server cluster or distributed file system composed of a plurality of physical servers, or at least one of the cloud servers providing basic cloud computing services such as cloud services, cloud databases, cloud computing, cloud functions, cloud storage, network services, cloud communication, middleware services, domain name services, security services, content delivery networks, as well as big data or artificial intelligence platforms, which is not limited in the embodiments of the present disclosure. In some embodiments, a number of servers mentioned above may be more or less, which is not limited in the embodiments of the present disclosure. Of course, the server may further include other functions to provide more comprehensive and diverse services.

In some embodiments, the proximal device 103 may run a computer system including a processor for implementing the target volume positioning method provided in the embodiments of the present disclosure.

In some embodiments, as shown in FIG. 4, the target volume positioning system provided in the embodiments of the present disclosure may further include a remote device 104. The remote device 104 may communicate with the proximal device 103 and is configured with a second target volume positioning model.

Herein, the second target volume positioning model is configured to determine the dynamic mapping relationship for the target volume based on a plurality of kinds of modalities of medical image data for the target object obtained during a positioning stage.

In some embodiments, the plurality of kinds of modalities of medical image data include body surface optical data and at least one of the following:
an X-ray projection image, a computed tomography (CT) image, a magnetic resonance imaging (MRI) image, a positron emission tomography (PET) image, a PET-CT image, a PET-MRI image, a single photon emission computed tomography (SPECT) image, a SPECT-CT image, an ultrasound imaging (US) image, a digital subtraction angiograph (DSA) image, a pathological slice image.

It should be noted that the plurality of kinds of modalities of medical image data may not necessarily be a positioning image acquired during the positioning stage, but may be image data acquired before the positioning stage, such as during the diagnostic stage. The emphasis in the positioning stage here is that the dynamic mapping relationship for the target volume is established in the positioning stage.

In some embodiments, the second target volume positioning model is further configured to: before performing the irradiation on the target volume of the target object, determine the target volume according to the plurality of kinds of modalities of medical image data for the target object; and generate an irradiation plan for the target volume according to the target volume.

In some embodiments, the second target volume positioning model may further be configured to process complex plurality of kinds of modalities of medical image data (such as CT images, PET images, MRI images, surface optical data, etc.) in a pre-treatment stage. Through high-dimensional feature extraction and deep learning, an individualized "biological target volume global feature library" of the patient may be constructed to achieve high-precision target volume modeling and knowledge expression.

In some embodiments, the second target volume positioning model is further configured to acquire irradiation data after the irradiation on the target volume is completed, and update based on the irradiation data.

In some examples, after the proximal device 103 finishes performing irradiation on the target volume, it may feed irradiation data back to the remote device 104. Correspondingly, the proximal device 103 receives the irradiation data fed back from the remote device 104.

Herein, the irradiation data may include at least one of the following: abnormal information during a process of performing the irradiation on the target volume; adjustment information for an irradiation strategy during the process of performing the irradiation on the target volume; or a correspondence between a position offset and an irradiation dose for the target volume during the process of performing the irradiation on the target volume.

In the embodiments of the present disclosure, the first target volume positioning model may be obtained by performing knowledge distillation on the second target volume positioning model. The first target volume positioning model may have the key features and core functions of the second target volume positioning model. A number of model parameters in the first target volume positioning model is smaller than a number of model parameters in the second target volume positioning model. Compared with the second target volume positioning model, the first target volume positioning model has the advantages of low training and deployment costs, and fast inference speed.

Herein, the knowledge distillation may include one or more of medical image data distillation, model parameter distillation, irradiation strategy distillation, etc.

In the embodiments of the present disclosure, the functions of the second target volume positioning model include: extracting global features from the plurality of kinds of modalities of medical image data, and identifying and positioning the target volume based on the extracted global features. Correspondingly, the functions of the first target volume positioning model may include: in real-time scenarios, quickly positioning the target volume based on image data of the target volume.

In some embodiments, the remote device 104 may be a computer device with a graphical user interface, and the computer device includes: one or more processors, a memory, and one or more application programs.

In the embodiments of the present disclosure, an entity of the remote device 104 may be a terminal or a server with a display, which is not limited in the embodiments of the present disclosure.

In some embodiments, the above-mentioned terminal may be at least one of devices such as a smartphone, a smart watch, a desktop computer, a laptop, a virtual reality terminal, an augmented reality terminal, a wireless terminal, or a portable laptop.

In some embodiments, the above-mentioned server may be an independent physical server, or a server cluster or distributed file system composed of a plurality of physical servers, or at least one of the cloud servers providing basic cloud computing services such as cloud services, cloud databases, cloud computing, cloud functions, cloud storage, network services, cloud communication, middleware services, domain name services, security services, content delivery networks, as well as big data or artificial intelligence platforms, which is not limited in the embodiments of the present disclosure. In some embodiments, a number of servers mentioned above may be more or less, which is not limited in the embodiments of the present disclosure. Of course, the server may further include other functions to provide more comprehensive and diverse services.

In some embodiments, the remote device 104 may run a computer system including a processor for implementing the target volume positioning method provided in the embodiments of the present disclosure.

In some embodiments, the above-mentioned target volume positioning system may also include a positioning device. The positioning device may be configured to perform simulated positioning on the target object before the irradiation is about to be performed on the target volume of the target object.

For example, the positioning device may perform simulated positioning based on image data of the target object. For example, before performing radiation therapy on the target volume of the target object, image acquisition may be performed on the target volume of the target object to obtain image data of the target volume. For example, image acquisition may be performed using at least one device among a computed tomography (CT) device, an emission computed tomography (ECT) device, a magnetic resonance imaging (MRI) device, a PET device, and an ultrasound examination device.

Furthermore, after obtaining the model positioning of the target volume, the positioning device may determine an irradiation plan for the target volume of the target object through a radiation therapy planning device.

In some embodiments, the radiation therapy planning device may include a TPS client and a TPS server. Among them, the TPS client may be at least one of devices such as smartphones, smart watches, desktop computers, laptops, virtual reality terminals, augmented reality terminals, wireless terminals, and portable laptops. For example, in some embodiments, the radiation therapy planning system run by the user on the TPS server through the TPS client may trigger the TPS server to execute the adaptive irradiation plan optimization process and display the optimized irradiation plan. In this way, users' time may be effectively saved and the optimized irradiation plan may be presented more intuitively, so that the users may evaluate the irradiation plan.

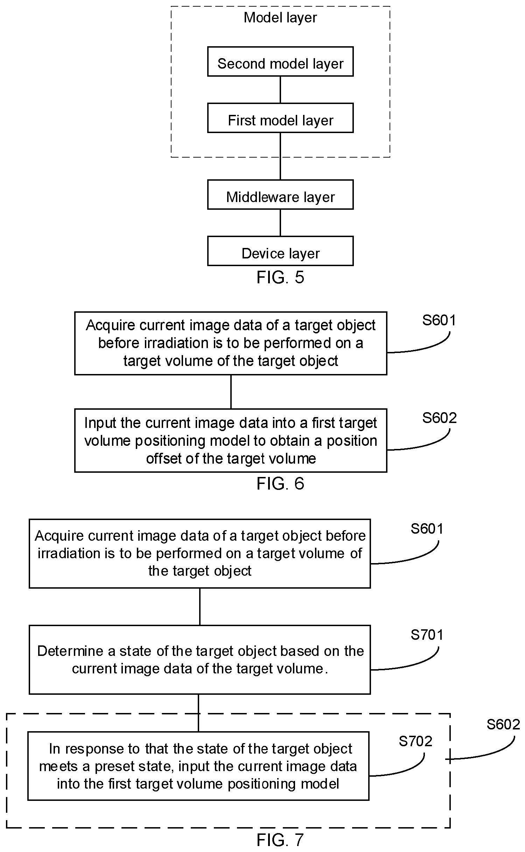

FIG. 5 is a schematic diagram of another target volume positioning system provided in the embodiments of the present disclosure, and the target volume positioning system may include: a model layer, a middleware layer, and a device layer. Below are explanations of these layers.

### 1-1. Model layer

Herein, the model layer may include a first model layer and a second model layer. The first model layer may be deployed on the above-mentioned proximal device 103, and the first model layer may be configured with the above-mentioned first target volume positioning model. The second model layer may be deployed on the above-mentioned remote device 104, and the second model layer may be configured with the above-mentioned second target volume positioning model. The first model layer may acquire the current image data of the target object (such as a patient), and combine the knowledge of the second target volume positioning model to quickly perform positioning on the target volume, and output the indication information on whether to perform irradiation. The second model layer may fuse the plurality of kinds of modalities of medical image data of the target object, and output a core feature (knowledge) with distillation to the first model layer.

In the embodiments of the present disclosure, the second model layer may further be used for global learning, feature extraction, knowledge distillation, target modeling, etc., on the plurality of kinds of modalities of medical image data. The first model layer may also be used for real-time reasoning (such as determining whether to perform radiation therapy based on an original irradiation plan), dynamic adjustment of radiation therapy position and/or dose, target volume positioning, dose optimization, etc.

Herein, the second model layer may optimize the complex features and knowledge of the second target volume positioning model through knowledge distillation technology, resulting in the first target volume positioning model. In this way, the first target volume positioning model may have a core capability of the second target volume positioning model, but the computational and model parameter quantities are significantly reduced.

In the embodiments of the present disclosure, the first target volume positioning model may feed the abnormal data and irradiation data generated during the real-time reasoning process back to the second target volume positioning model when the proximal device is running, so that the second target volume positioning model may be updated based on the abnormal data and irradiation data fed back from the first target volume positioning model.

Based on the collaborative work between the first target volume positioning model and the second target volume positioning model, a balance between high accuracy and high real-time performance may be achieved. On the one hand, it meets the precision requirements of precision radiation therapy, and on the other hand, it also ensures the real-time and stability of the treatment process.

### 1-2. Device layer

Herein, the device layer may be deployed in devices close to the target object, such as in the image acquisition device 101 and the radiation therapy device.

In a case where the device layer is deployed in the image acquisition device 101, the device layer may be configured to acquire the plurality of kinds of modalities of medical image data of the target object and send the collected plurality of kinds of modalities of medical image data to the model layer. The device layer may also be configured to collect image data of the target object in real time and send the collected image data to the model layer.

In a case where the device layer is deployed in the radiation therapy device, the device layer may execute indication information from the model layer and feed irradiation data and device operation data back to the model layer.

Furthermore, in order to ensure the normal transmission of data between the device layer and the model layer, as shown in FIG. 4, the target volume positioning system provided in the embodiments of the present disclosure may also include:

### 1-3. Middleware layer

Herein, the middleware layer may be deployed on the proximal device 103, or on the image acquisition device 101 or radiation therapy device. Of course, the middleware layer may be deployed on an independent device, and the independent device is located between a device deployed at the model layer and a device deployed at the device layer. For example, it may be deployed between the proximal device 103 and the image acquisition device 101.

In the embodiments of the present disclosure, the middleware layer may be configured to implement data transmission between the model layer and the device layer. For example, the middleware layer may forward indication information from the model layer to the device layer, as well as image data, irradiation data, device operation data, patient breath holding status information, etc., from the device layer to the model layer.

Herein, the indication information may include a motion adjustment instruction for the radiation therapy device, a dose distribution adjustment instruction, a breath holding control instruction, etc. The motion adjustment instruction for the radiation therapy device may be used to adjust a position of radiation therapy device. The dose distribution adjustment instruction may be used to indicate the radiation therapy device to adjust an irradiation dose for different areas of the target volume. The breath holding control instruction may be used to indicate that a patient needs to be in a breath holding state when the irradiation is performed on the target volume. Breath holding state information for the patient may include a respiratory state of the patient, whether the patient is in a breath holding state, or the like. A running state of a device may include whether the device is running normally.

In the embodiments of the present disclosure, the device where the model layer is located and the device where the device layer is located support different data transmission protocols. The middleware layer may perform format conversion on the received data/information and forward the format converted data/information. In this way, it may ensure that the device where the model layer is located and the device where the device layer is located may perform data transmission normally. Based on the system architecture in FIG. 5, the combination of high precision (the function of the second model layer) and high real-time (the function of the first model layer) may be achieved. At the same time, the middleware layer realizes the decoupling between the model layer and the device layer, ensuring the flexibility and scalability of the system.

The target volume positioning system in the embodiments of the present disclosure is introduced above.

The target volume positioning method in the embodiments of the present disclosure may be applied to the above-mentioned target volume positioning system. A detailed explanation for the target volume positioning method may be provided below.

It should be noted that the target volume positioning method provided in the embodiments of the present disclosure may be applied to a scenario of each fractionated radiation therapy in in a plurality of fractionated radiation therapies, as well as to a scenario of single radiation therapy.

FIG. 6 is a flowchart of a target volume positioning method provided in the embodiments of the present disclosure. The target volume positioning method provided in the embodiments of the present disclosure may be applied to a proximal device configured with a first target volume positioning model. As shown in FIG. 6, the method includes the following S601-S602.

In S601, current image data of a target object is acquired before irradiation is to be performed on a target volume of the target object.

Herein, the target object may be a patient to be performed with radiation therapy or a phantom of a simulated patient model. The target volume may be a region on the target object that requires to be performed with irradiation. The current image data of the target object may include body surface optical data and/or an X-ray projection image.

In one implementation, the proximal device may acquire the current image data of the target object through an image acquisition device before performing the irradiation on the target volume of the target object.

In S602, the current image data is input into a first target volume positioning model to obtain a position offset of the target volume.

Herein, the position offset of the target volume may refer to an offset between a current position of the target volume and a reference position of the target volume (such as a position of the target volume in the irradiation plan). The current position of the target volume may be determined based on the current image data of the target object. Specifically, the description of the following embodiments may be referred.

In one implementation, the first target volume positioning model is configured to determine the position offset of the target volume based on a dynamic mapping relationship for the target volume after acquiring the current image data of the target object.

Herein, the dynamic mapping relationship for the target volume may be: a correspondence between a position of the target volume and the image data with motion changes of the target volume. The motion changes of the target volume may refer to changes in the position of the target volume caused by physiological activities of the target object (such as coughing, breathing, and other non-autonomous movements). The motion changes may include both non-periodic motion and periodic motion. The non-periodic motion may include conscious motion such as coughing and convulsions, while the periodic motion may include uncontrolled motion such as breathing, heartbeat, pulse, and gastrointestinal peristalsis.

Based on the technical solutions in FIG. 6, before performing irradiation on the target volume of the target object, the current image data of the target volume may be input into the first target volume positioning model, so that the first target volume positioning model may determine the position offset of the target volume based on the dynamic mapping relationship for the target volume. Due to the dynamic mapping relationship for the target volume is a correspondence between the position of the target volume and the image data for changes with the motion of the target volume, therefore the position offset of the target volume determined based on the dynamic mapping relationship takes into account the impact of changes in the patient's physiological activities. Furthermore, based on the position offset of the target volume, whether to perform the irradiation on the target volume and an irradiation position in the future may be accurately determined.

It should be noted that the target volume in the above embodiments may be a biological tumor target volume.

In some embodiments, the method provided in the embodiments of the present disclosure may further include: acquiring a radiation therapy guidance image of the target object during a process of acquiring the current image data of the target object.

Herein, the radiation therapy guidance image may include the target volume inside the target object. The radiation therapy guidance image has the features of low dose and fast imaging.

Furthermore, in S602, the proximal device may input the current image data of the target object and the radiation therapy guidance image into the first target volume positioning model to obtain the position offset of the target volume.

Here, the first target volume positioning model also has fusion/registration function, which may fuse or register the current image data of the target object and the radiation therapy guidance image. For example, in a case where the current image data is body surface optical data and the radiation therapy guidance image is an X-ray projection image, the first target volume positioning model may register the current body surface optical data of the target object with the current X-ray projection image.

Based on this embodiment, since the radiation therapy guidance image of the target object may reflect the position information of the target volume inside the target object, there is more tissue information based on the current image data of the target object and the radiation therapy guidance image, thus the target volume may be positioned more accurately.

In some embodiments, as shown in FIG. 7, the method provided in the embodiments of the present disclosure may further include: S701 and S702.

In S701, a state of the target object is determined based on the current image data of the target volume.

Herein, the state of the target object may include an abnormal motion state and/or a breath holding state. The abnormal motion state may include sudden body motion, an abnormal respiratory rate, and other abnormal motion states of the patient. A normal motion state may include normal breathing, heartbeat, and other states.

In one implementation, the current image data include body surface optical data. The proximal device may detect whether the patient is in an abnormal motion state in real time based on the body surface optical data.

In the embodiments of the present disclosure, the body surface optical data may capture an amplitude, a period, and a phase of a respiratory motion in real time, providing indirect but effective reference for organ motion. That is to say, the proximal device may detect the patient's state based on the body surface optical data.

Furthermore, the above S602 may be implemented through S702.

In S702, in response to that the state of the target object meets a preset state, the current image data is input into the first target volume positioning model.

Herein, the preset state may include a state with no abnormal motion, that is, a state in which the target object has not undergone abnormal motion; the preset state may also include a breath holding state without an abnormal motion state.

Based on the technical solutions in FIG. 7, after acquiring the current image data of the target object, a current state of the target object may be determined based on the current image data. In a case where the target object is in the preset state, it indicates that the position of the target volume may not be affected by the abnormal motion of the target object. At this time, the current image data may be input into the first target volume positioning model. Thus, the position offset of the target volume may be more accurately determined.

In some embodiments, after determining the position offset of the target volume, the first target volume positioning model provided in the embodiments of the present disclosure is further configured to: in response to that the position offset of the target volume is within a range of a preset threshold, output first indication information for indicating to perform the irradiation on the target volume; in response to that the position offset of the target volume is not within the range of the preset threshold, output second indication information for indicating to adjust the position of the target volume or third indication information for indicating to terminate to perform irradiation.

Herein, the range of the preset threshold may be set as needed, for example, it may be 0-1 millimeters. That is to say, in a case where the position offset of the target volume is less than or equal to 1 millimeter, the first indication information may be output. In a case where the position offset of the target volume exceeds 1 millimeter, the second indication information may be output.

It could be understood that in a case where the position offset of the target volume is not within the range of the preset threshold, after adjusting the position of the target volume based on the second indication information, the first target volume positioning model may also re-determine the position of the target volume until the adjusted offset of the target volume is within the range of the preset threshold. The radiation therapy device may then perform irradiation on the re-determined position of the target volume based on the first indication information.

For example, the first target volume positioning model may determine a current position of the target volume based on a dynamic adjustment rule for the target volume. Herein, the dynamic adjustment rule for the target volume may be: a rule for the position offset of the target volume and changes of parameters of a shape and/or position of the target volume.

Based on the technical solutions of this embodiment, when the position offset of the target volume is within the range of the preset threshold, it indicates that the position offset of the target volume is within an acceptable range, that is, the irradiation may be performed on the target volume according to the current irradiation plan. When the position offset of the target volume is not within the range of the preset threshold, it indicates that the position offset of the target volume is relatively large. If irradiation is still performed on the target volume according to the current irradiation plan, it may lead to poor radiation therapy effect. Therefore, by adjusting the position of the target volume, precise irradiation for the target volume may be ensured in the future.

Furthermore, the first target volume positioning model provided in the embodiments of the present disclosure is specifically configured to: in response to that the position offset of the target volume is within a range of a first threshold, output first indication information for indicating to perform the irradiation on the target volume; in response to that the position offset of the target volume is not within the range of the first threshold and is within a range of a second threshold, output second indication information for indicating to adjust the position of the target volume; in response to that the position offset of the target volume exceeds the range of the second threshold, output third indication information for indicating to terminate to perform irradiation. Herein, the range of first threshold is greater than or equal to the first threshold and less than or equal to the second threshold, and the range of the second threshold is greater than the second threshold and less than a third threshold. For example, the range of the first threshold may be [0 mm, 1 mm], and the range of the second threshold may be (1 mm, 3 mm]. In this way, a situation where irradiation continues to be performed even when there is a significant offset in the target volume, which could affect the effectiveness of radiotherapy, may be avoided.

In some embodiments, in order to ensure the accuracy of irradiation, the method provided in the embodiments of the present disclosure may further include: in response to that a time interval between a acquisition time for the current image data and an outputting time for the first indication information is shorter than a preset time interval, the proximal device may control a radiation therapy device to perform the irradiation on the target volume.

Herein, the preset time interval may be in a millisecond range. For example, it may be any value between 20 milliseconds and 50 milliseconds, such as 20 milliseconds, 30 milliseconds, 50 milliseconds, etc.

In one example, the proximal device may determine the time interval between the acquisition time for the current image data and the outputting time for the first indication information based on a timestamp carried by the current image data and a timestamp for generating the first indication information.

In this way, due to a short time interval between an image acquisition time of the image acquisition device and the time indicated for performing irradiation on the target volume, which is in the millisecond range, and during this time duration, the target volume may not experience significant position offset, so the radiation therapy device may accurately perform irradiation on the target volume.

In some embodiments, the method provided in the embodiments of the present disclosure may further include: in response to the target object is in a breath holding state without an abnormal motion state, performing the irradiation on the target volume. Due to that the target object is in the breath holding state without an abnormal motion state, the motion of the target volume inside the target object is limited. In this way, the irradiation beam may be more accurately irradiated onto the target volume, improving the accuracy of radiation therapy.

In one example, combined with the embodiments shown in FIG. 7 above, the proximal device may determine whether the target object is in a breath holding state and a motion state based on the current image data of the target object.

In one implementation, in a case where the position offset of the target volume is within the range of the preset threshold, and if the target object is in a breath holding state without an abnormal motion state, the proximal device may send the first indication information to the radiation therapy device. After the radiation therapy device receives the first indication information, the radiation therapy device may perform irradiation on the target volume in response to the first indication information.

In another implementation, the proximal device may be configured with a state detection model, the state detection model may be configured to detect whether the target object is in a breath holding state and whether the target object is in an abnormal motion state. Herein, the state detection model may be the first target volume positioning model or a different model from the first target volume positioning model.

In one example, the proximal device may input the current image data of the target object into the state detection model, and the state detection model is configured to detect whether the target object is in a breath holding state and whether the target object is in an abnormal motion state.

If the target object is in a breath holding state and the position offset of the target volume does not exceed the range of the preset threshold, the irradiation may be performed on the target volume. If the patient is not in a breath holding state and/or the patient is in an abnormal motion state, the irradiation for the target volume may be paused, or an alarm message indicating abnormal breath holding state of the patient may be output.

Furthermore, during the irradiation process on the patient, real-time monitoring of whether the irradiation is performed within the breath holding window is also possible. If it is in the breath holding window, the irradiation may be performed on the target volume; if it is not within the breath holding window or exceeds the breath holding window, the irradiation for the target volume may be paused.

Based on the technical solutions of this embodiment, in a case where the target object is in a breath holding state without an abnormal motion state, the position of the target volume is basically not subject to offset. Therefore, the proximal device may perform irradiation on the target volume, ensuring precise irradiation of the target volume.

In some embodiments, the method provided in the embodiments of the present disclosure may further include: acquiring the dynamic mapping relationship for the target volume.

Herein, the dynamic mapping relationship for the target volume is determined by a second target volume positioning model based on the plurality of kinds of modalities of medical image data for the target object acquired during a positioning stage.

In the embodiments of the present disclosure, a dynamic position of the target volume may be quickly determined through the image data of the target volume and the body surface optical data of the target object. Meanwhile, due to the fact that body surface optical data may compensate for the insufficient temporal resolution of image data, it may provide real-time and continuous dynamic information of the target volume.

In some embodiments, the first target volume positioning model may be obtained by performing knowledge distillation for the second target volume positioning model.

Herein, the knowledge distillation may include at least one of: medical image data distillation, model parameter distillation, or irradiation strategy distillation. The medical image data distillation may refer to: extracting intermediate features (such as the fused features of plurality of kinds of modalities of medical images) of the second target volume positioning model, and optimizing them through feature matching and loss function. The model parameter distillation may refer to: compressing key model parameters (such as a registration matrix and a motion mapping function) of the second target volume positioning model into a format that is usable for the first target volume positioning model. The irradiation strategy distillation may refer to: converting a strategy of the second target volume positioning model (such as a dose optimization strategy) into lightweight rules for use by the first target volume positioning model.

Based on the technical solutions of this embodiment, since the first target volume positioning model is obtained by performing knowledge distillation for the second target volume positioning model, that is, the first target volume positioning model has the functions of the second target volume positioning model, and a size of the first target volume positioning model is small, the proximal device may quickly and accurately position the target volume based on the first target volume positioning model without consuming a lot of storage resources.

In some embodiments, the second target volume positioning model may further be configured to: before performing the irradiation on the target volume of the target object, determine the target volume according to the plurality of kinds of modalities of medical image data for the target object; and generate an irradiation plan for the target volume according to the target volume.

Herein, the irradiation plan includes: a dynamic position adjustment plan for the target volume and a dose distribution plan. The plurality of kinds of modalities of medical image data include body surface optical data and an imaging image. The imaging image may include at least one of: an X-ray projection image, a computed tomography (CT) image, a magnetic resonance imaging (MRI) image, a positron emission tomography (PET) image, a PET-CT image, a PET-MRI image, a single photon emission computed tomography (SPECT) image, a SPECT-CT image, an ultrasound imaging (US) image, a digital subtraction angiograph (DSA) image, a pathological slice image.

In some embodiments, the method provided in the embodiments of the present disclosure may further include: after the irradiation on the target volume is completed, the proximal device may feed irradiation data back to the second target volume positioning model, so that the second target volume positioning model is updated based on the irradiation data. Alternatively, the proximal device may feed the irradiation data back to the remote device, and upon receiving the irradiation data, the remote device may update the second target volume positioning model based on the irradiation data.

Herein, the irradiation data may include at least one of the following.

### 2-1. Abnormal information during a process of irradiation

Herein, the abnormal information may be used to indicate/include: abnormal respiratory state of the target object (such as excessive patient breathing rate), abnormal operation of radiation therapy device, abnormal current image data of the target volume, abnormal morphology of the target volume, and incomplete coverage of one or more of the target volumes by the irradiation area.

### 2-2. Adjustment information for an irradiation strategy during the process of irradiation

Herein, the adjustment information for an irradiation strategy may include an adjusted position of the target volume, an irradiation dose for the target volume, etc.

In one example, in a case where the position offset of the target volume is not within the range of the preset threshold, due to the offset of the target volume, the proximal device may reposition the target volume based on the current image data of the target volume. Meanwhile, the offset of the target volume may also lead to changes in the shape of a heterogeneous region and a boundary region of the target volume. Due to the different irradiation doses corresponding to different regions, the proximal device may readjust the irradiation dose for each region based on shape of different regions in the target volume.

### 2-3. Correspondence between a position offset and an irradiation dose for the target volume during the process of the irradiation

Herein, the correspondence between the position offset and the irradiation dose may refer to an adjusted irradiation dose after the position of the target volume is adjusted. That is to say, after controlling the radiation therapy device to perform irradiation on the target volume, the proximal device may obtain the position offset of the target volume and an irradiation dose corresponding to the position offset used during radiation therapy, thus obtaining the correspondence between the position offset and the irradiation dose for the target volume.

In the embodiments of the present disclosure, during the process of updating the second target volume positioning model, the first target volume positioning model may also be updated. For example, the first target volume positioning model and the second target volume positioning model may be updated based on federated learning.

In one example, after the remote device updates the second target volume positioning model, the remote device may perform knowledge distillation on updated model parameters, and send the distilled model parameters to the proximal device, so that the proximal device may be updated based on the distilled model parameters.

Based on the technical solutions of this embodiment, after completing radiation therapy on the target volume, the proximal device may feed irradiation data back to the second target volume positioning model, so that the second target volume positioning model may be updated based on the irradiation data, ensuring the accuracy of the second target volume positioning model.

One implementation is introduced in combination with the above-mentioned entire radiation therapy process.

In a positioning stage, a plurality of kinds of modalities of medical image data of the target object may be acquired through a positioning device, and the plurality of kinds of modalities of medical image data include body surface optical data and at least one of the following:
an X-ray projection image, a CT image, an MRI image, a PET image, a PET-CT image, a PET-MRI image, an SPECT image, a SPECT-CT image, an US image, a DSA image, a pathological slice image.

In a plan design and verification stage, the second target volume positioning model may acquire a plurality of kinds of medical image data of the target object, and fuse the plurality of kinds of medical image data of the target object, determine the target volume, and generate the dynamic mapping relationship for the target volume of the target object; generate an irradiation plan based on the target volume and validate the irradiation plan generated by the second target volume positioning model. Based on the technical solutions of this embodiment, since the medical image data of the target object may reflect the position of the target volume, the fusion of the plurality of kinds of medical image data of the target object may more accurately position the target volume. In this way, the irradiation plan for the target volume may also be more precise.

In a radiation therapy stage, before irradiation is about to be performed on the target volume of the target object, the first target volume positioning model acquires the dynamic mapping relationship for the target volume of the target object, and uses the image acquisition device to acquire the current image data of the target object (such as body surface optical data and/or X-ray projection images). The current image data is input into the first target volume positioning model to determine the position offset of the target volume based on the dynamic mapping relationship for the target object.

The following may provide a detailed explanation of the target volume positioning method involved in the present disclosure based on specific embodiments.

With the rapid development of precision medicine, tumor radiation therapy is gradually transitioning from a traditional "anatomy driven" mode to a "biology driven" mode. Herein, the "anatomy driven" mode refers to the precise control of dose distribution based on the structure of the tumor through physical techniques. The "biology driven" mode refers to determining individualized treatment plans based on biological features of tumors, combined with molecular markers and functional influences.

However, in clinical practice, the delineation technology for a tumor target volume still faces multiple bottlenecks and urgently needs technological innovation and breakthroughs. On the one hand, the traditional delineation for the target volume mainly relies on a single image (such as a CT image, a PET image, etc.), which is difficult to fully integrate structural, metabolic, and functional information of the tumor, resulting in blurred target volume boundaries and insufficient identification of heterogeneous regions, directly affecting the accuracy and efficacy of treatment. On the other hand, traditional target volume delineation heavily relies on manual annotation, which is limited by the subjective experience and operating habits of doctors. Under dynamic physiological states (such as coughing, involuntary position changes, breathing, gastrointestinal peristalsis, heart beats, etc.), target volume recognition has significant inconsistencies and errors, making it difficult to meet the individualized needs of complex cases.

In addition, precision radiation therapy imposes strict requirements on device real-time responsiveness, algorithm efficiency, and cross platform compatibility. Existing artificial intelligence assisted decision-making systems rely heavily on large cloud models, which suffer from communication delays, poor adaptability to network outages, and insufficient hardware computing power, and cannot meet the real-time and reliability requirements in dynamic treatment scenarios. Therefore, the accuracy and safety of radiation therapy face challenges.

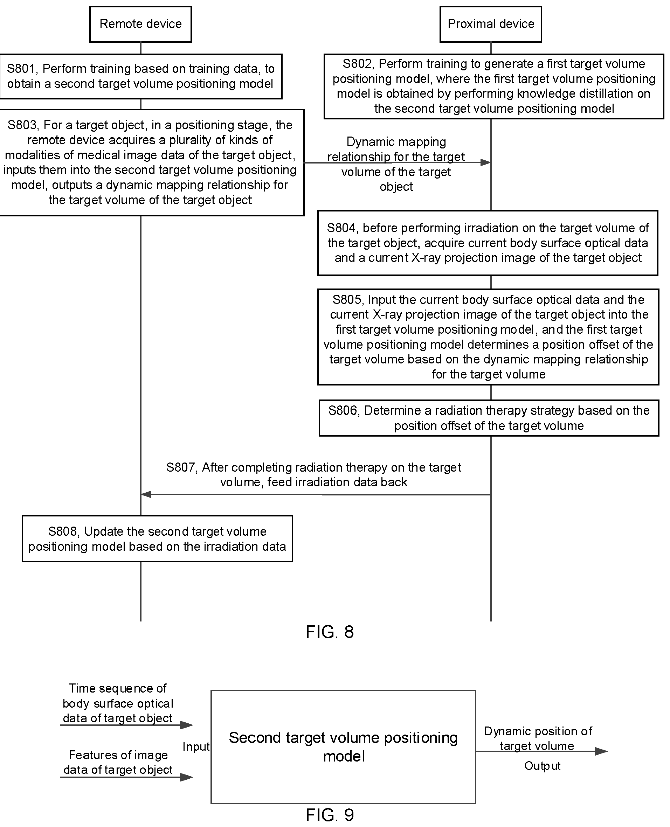

In view of this, the precise recognition of the target volume will be achieved through the collaborative cooperation of the first target volume positioning model and the second target volume positioning model. Taking the current image data as body surface optical data and X-ray projection image as examples, the target volume positioning method may be explained. As shown in FIG. 8, the method includes S801-S808.

In S801, a remote device performs training based on training data, to obtain a second target volume positioning model.

Herein, the training data includes a plurality of kinds of modalities of medical image data of a plurality of objects, and correspondences between positions of target volumes of different objects change and image data as the target volumes move.

Here, the plurality of kinds of modalities of medical image data include body surface optical data and at least one of: an X-ray projection image, a CT image, an MRI image, a PET image, a PET-CT image, a PET-MRI image, an SPECT image, a SPECT-CT image, an ultrasound (US) image, a DSA image, and a pathological slice image.

Herein, the body surface optical data mainly reflects dynamic changes of the patient's external body surface. In radiation therapy scenarios, there is an association relationship between the body surface optical data and the motion of the patient's organs. For example, in radiation therapy for abdominal tumors (such as a tumor in lung or a tumor in liver), the patient's respiratory motion may cause the target volume to move. And the body surface optical data may be obtained in real time with organ motion data such as an amplitude, a period, and a phase of respiratory motion, etc.

For example, due to the non-invasive nature of the body surface optical data, it may not cause additional damage to patients and has a fast collection speed. Therefore, real-time monitoring for the target volume may be implemented through body surface optical data.

For example, in breath holding radiation therapy, surface optical data may be used to monitor in real-time whether the patient maintains a breath holding state and the stability of the breath holding state, thereby providing feedback basis for irradiation.

In some instances, the body surface optical data may be used for position guidance and dynamic monitoring in radiation therapy. The body surface optical data may include displacement information of a marker point. The displacement information of a marker point may reflect the motion of internal organs.

In one implementation, the body surface optical data of a target object may be acquired through cameras (such as depth cameras, optical cameras) and scanners (such as 3D optical scanners, laser scanners).

In the embodiments of the present disclosure, the input of the trained second target volume positioning model is a plurality of kinds of modalities of medical image data of the target object, and the output is a dynamic mapping relationship for the target volume, and the dynamic mapping relationship for the target volume is: a correspondence between a position of the target volume of the target object and the image data with motion changes of the target volume. Specifically, a generation process of the second target volume positioning model may include the following steps.

### Step 1: data preparation

Herein, data preparation may include data annotation and data preprocessing. The data annotation refers to: it is necessary to standardize a true position of the target volume at a corresponding time point for each frame of body surface optical data and image data. Data preprocessing may include processing for optical surface data and processing for image data.

Specifically, the processing for surface optical data may include: denoising (filtering out highfrequency noise in optical signals), normalization (normalizing landmark point coordinates or point cloud data to a unified standard), and time alignment (aligning the time points of surface optical data with image data). The processing of image data may include: image segmentation (extracting regions of the target volume), feature extraction (extracting image features through pre-set deep learning models). Deep learning model may include ResNet and UNet.

### Step 2: model design

Herein, model design refers to selecting a suitable initial model or algorithm as the model architecture for the second target volume positioning model.

In the embodiments of the present disclosure, a deep learning model may be used as the model architecture for the second target volume positioning model.

### Step 3: model training

Herein, a loss function used for model training may be a mean square error, which measures a error between a predicted position and a true position of the target volume.

During the training process of the model, the training data may also be enhanced, the model may be optimized, and evaluated to obtain the trained second target volume positioning model.

Herein, the data enhancement may include: enhancement for the body surface optical data and enhancement for the image data. The enhancement for the body surface optical data may include: adding random noise to the body surface optical data to simulate an error in actual acquisition; the random truncation or extension for a time sequence of the body surface optical data enhances the robustness of the model.

An optimization manner for the model may be: using Adam optimizer or other optimization algorithms suitable for deep learning. A learning rate may be dynamically adjusted (such as using a learning rate scheduler).

The model evaluation may be done by using a validation set to assess the predictive accuracy of the model. Evaluation metrics may include: a mean absolute error (MAE), a root mean square error (RMSE), and a correlation of dynamic trajectories (such as a DTW distance), etc.

In S802, a proximal device performs training to generate a first target volume positioning model, where the first target volume positioning model is obtained by performing knowledge distillation on the second target volume positioning model.

Herein, a result of performing the knowledge distillation results on the second target volume positioning model may include: a fusion rule for the plurality of kinds of modalities of medical image data, boundaries and heterogeneous regions of a target volume, a dose optimization strategy, an anomaly detection model and adjustment rules, and dynamic mapping relationship for the target volume.

In the process of knowledge distillation, the second target volume positioning model may also adjust the weights of different modal features according to scenario requirements. For example, in a respiratory sensitive scenario, priority is given to distilling the body surface optical data and a CT image. In a heterogeneous region recognition scenario, priority is given to distilling a PET image and an MRI image.

In the embodiments of the present disclosure, the second target volume positioning model may also transfer key information for distillation to the first target volume positioning model. Herein, the key information may include global features and a dynamic motion model for the target volume, such as a dynamic mapping relationship for the target volume. The global features for the target volume may include its anatomical boundaries, metabolic hotspots, functional zoning, and heterogeneous regions. Correspondingly, the distillation content of the global features for the target volume may include: spatial features (such as precise contours of tumor anatomical structures in a CT image), metabolic features (such as SUV value distribution in a PET image, reflecting metabolic activity regions), functional features (such as a DWI (diffusion-weighted imaging) and T2 weighted image in an MRI image, capturing functional zones within the tumor), heterogeneity features (such as high-risk sub regions extracted in combination with the plurality of kinds of modalities of medical image data).

The dynamic motion model (dynamic mapping relationship for target volume) may include: a mapping relationship between the patient's body surface motions (such as respiratory motions) and internal organ displacements. Correspondingly, the distillation content of the dynamic motion model may include: motion feature mapping function (a dynamic mapping relationship between a body surface optical marker point and an internal target displacement generated by training by the second target volume positioning model), breath holding state features (such as a target volume position reference and motion pattern under a breath holding state), and time sequence features (such as a dynamic displacement pattern during respiratory cycle).

In one example, the second target volume positioning model may also be configured to: align the plurality of kinds of modalities of medical image data in features, and perform knowledge distillation on the aligned plurality of kinds of modalities of medical image data.

Herein, feature alignment may refer to: aligning a plurality of kinds of modalities of medical image data such as CT, MRI, PET, etc., spatially and semantically to ensure feature consistency. Correspondingly, the distillation content of the plurality of kinds of modalities of medical image data may include registration parameters (spatial registration parameters among the plurality of kinds of modalities of medical image data, such as rotation, translation, and scaling matrices), feature alignment weights (a weight allocation strategy for cross modal feature fusion, such as CT prioritizing anatomical structures and PET prioritizing metabolic hotspots).

In another example, the second target volume positioning model may further be configured to: generate a target volume feature model based on individualized data of the target object, and perform knowledge distillation on the target volume feature model.

Herein, the distillation content of the target volume feature model may include: individualized target volume template (a target volume reference model generated for the target object before treatment), target volume dynamic adjustment rules (parameterized rules for adjusting the shape and position of the target volume based on the current image data of the target object).

During the treatment process, the second target volume positioning model may use the target volume feature template of the target object as a reference, and dynamically adjust it based on the current image data of the target object to ensure that the model parameters of the first target volume positioning model are always consistent with the actual state of the target object.

In another example, the second target volume positioning model may also generate an irradiation plan and perform knowledge distillation on the irradiation plan.

Herein, the irradiation plan may include a dose distribution optimization strategy. For example, it may include: an optimization plan for dose distribution in the target volume, thus maximizing therapeutic efficacy and protecting normal tissues. Correspondingly, the distillation content of the irradiation plan may include: a dose distribution template (such as an individualized dose distribution plan generated based on tumor heterogeneity features), dose adjustment rules (real-time adjustment of irradiation regions based on dynamic changes in the target volume).

In S803, for a target object, in a positioning stage, the remote device acquires a plurality of kinds of modalities of medical image data of the target object, inputs them into the second target volume positioning model, outputs a dynamic mapping relationship for the target volume of the target object, and sends the dynamic mapping relationship for the target volume of the target object to the proximal device. Correspondingly, the proximal device receives the dynamic mapping relationship for the target volume of the target object from the remote device.

Herein, the plurality of kinds of modalities of medical image data include body surface optical data and at least one of: an X-ray projection image, a CT image, an MRI image, a PET image, a PET-CT image, a PET-MRI image, an SPECT image, a SPECT-CT image, an ultrasound (US) image, a DSA image, and a pathological slice image.

In the embodiments of the present disclosure, the second target volume positioning model may also fuse the body surface optical data with other types of high-quality of the plurality of kinds of modalities of medical image data to position the target volume.

For example, the second target volume positioning model may fuse the body surface optical data with other types of high-quality image data through the following methods.

### 3-1. Fusion based on marker points.

Herein, the fusion method based on marker points may be: placing optical marker points (such as reflective spheres or texture patterns) on the body surface of the patient, and calibrating the position of the optical marker points in the image data, so that the mapping relationship between the marker points in the body surface optical coordinate system and the image coordinates may be established, which is simple and intuitive, suitable for static or regular motion scenarios.

### 3-2. Fusion based on surface contour.

Herein, the fusion method based on surface contour may be: acquiring a three-dimensional contour of the patient's body surface through optical scanning device, registering it with the body surface contour in the image data, and using point cloud configuration algorithms (such as ICP algorithm) or surface matching algorithms to achieve contour alignment. This method may capture a large amount of body surface information and is suitable for complex body shape changes.

### 3-3. Fusion based on motion models.

Herein, the fusion method based on motion models may be: using body surface optical data to construct a patient's motion model (such as respiratory motion model), establishing a correlation model between organ position and surface motion through image data, and driving the motion model through surface optical data to predict the dynamic position of the target volume. Based on this method, real-time dynamic tracking of the target volume may be achieved, which is suitable for periodic motion such as respiratory motion.

### 3-4. Fusion based on deep learning.

Herein, the fusion method based on deep learning may be: using deep learning models such as convolutional neural networks and recurrent neural networks to jointly model body surface optical data and image data, the time sequence of surface optical data and the features of image data are input into the model to obtain the dynamic position of tumors. This method may handle complex nonlinear relationships, and is adapted to a plurality of motion modes with high degree of automation, thus reducing human intervention.

In one example, in the scenario of positioning the target volume, some deficiencies in medical image data may be compensated for through body surface optical data. Specifically, the body surface optical data has the following advantages.

### 4-1. Improving the dynamic accuracy of target volume positioning.

Herein, there is usually a certain correlation between the body surface optical data and organs. By combining the body surface optical data, the position of the target volume may be dynamically adjusted to avoid offset caused by organ motion. For example, in radiation therapy for a tumor in lung, the up and down motion of marker points in the body surface optical data may reflect the longitudinal displacement of the tumor in lung. For example, in radiation therapy for a tumor in liver, the lateral displacement of marker points in the body surface optical data may reflect the range of motion of the liver.

In addition, in clinical application scenarios such as radiation therapy, the stability of the patient's breath holding state may be monitored in real time through the body surface optical data, ensuring that irradiation is carried out within the breath holding window, thereby improving the accuracy of target volume positioning.

Furthermore, through the body surface optical data, the patient may be monitored in real-time for abnormal motions (such as sudden body motions or unstable breath holding states), which may trigger the suspension of irradiation or adaptive adjustment of irradiation plans in the event of abnormal motions.

### 4-2. Having good time resolution.

Herein, an acquisition time for medical image data is usually intermittent, while acquisition for the body surface optical data is continuous. Therefore, it is possible to acquire body surface optical data during an acquisition interval for medical image data, which may compensate for the problem of insufficient temporal resolution of medical images.

In S804, before performing irradiation on the target volume of the target object, the proximal device acquires current body surface optical data and a current X-ray projection image of the target object.

In S805, the proximal device inputs the current body surface optical data and the current X-ray projection image of the target object into the first target volume positioning model, and the first target volume positioning model determines a position offset of the target volume based on the dynamic mapping relationship for the target volume.

Herein, the first target volume positioning model may utilize the distilled knowledge of the second target volume positioning model to quickly predict the dynamic position of the target volume based on the body surface optical data, and adjust the irradiation direction and dose distribution of the radiation therapy device according to the dynamic position of the target volume.

Herein, the dynamic mapping relationship for the target volume may be: a correspondence between a position of the target volume of the target object and the image data with motion changes of the target volume.

In S806, the proximal device determines a radiation therapy strategy based on the position offset of the target volume.

Herein, the radiation therapy strategy may include continuing to perform irradiation on the target volume according to the original irradiation plan, adjusting the original irradiation plan and using the adjusted irradiation plan to perform irradiation on the re-positioned target volume, and stopping to perform irradiation on the target volume.

In one implementation, in a case where the position offset of the target volume is within a range of a preset threshold, the first target volume positioning model is configured to output first indication information for indicating to perform the irradiation on the target volume. That is to say, the first target volume positioning model may indicate to continue performing irradiation on the target volume according to the original irradiation plan.

In another implementation, in a case where the position offset of the target volume is not within the range of the preset threshold, the first target volume positioning model is configured to output second indication information for indicating to adjust the target volume or third indication information for indicating to terminate to perform irradiation.

In a case of outputting the second indication information, the first target volume positioning model may further be configured to adjust the original irradiation plan and re-position the target volume.

In some examples, the first target volume positioning model may determine whether the breath holding state of the target object meets irradiation requirements based on the current body surface optical data. If the irradiation requirements are not met, the irradiation may be terminated or the irradiation plan may be adjusted.

It should be noted that the first target volume positioning model runs on the proximal device and may directly use the lightweight features of the body surface optical data for fast inference, without the need for high computing power to process complex image data, thus improving data processing efficiency.

In S807, after completing radiation therapy on the target volume, the proximal device feeds irradiation data back to the remote device. Correspondingly, the remote device receives the irradiation data from the proximal device.

In S808, the remote device updates the second target volume positioning model based on the irradiation data.

Based on the technical solutions in FIG. 8, the remote device trains a plurality of kinds of modalities of medical image data to construct a second target volume positioning model (large model), and perform knowledge distillation on the large model to obtain a lightweight first target volume positioning model (small model), so that the proximal device may use this lightweight small model for real-time positioning of the target volume. That is to say, in the embodiments of the present disclosure, precise positioning of the target volume is achieved through the coordination and cooperation of the first target volume positioning model and the second target volume positioning model. At the same time, in dynamic treatment scenarios, real-time positioning of the target volume may be achieved with reliability.

It should be noted that in the embodiments of the present disclosure, the image data includes clinical data and laboratory data. The clinical data may include the body surface optical data (such as positions of marker positions, body surface contours) and image data acquired from the patient. The laboratory data may include simulated human models or data of healthy individuals acquired using a motion capture device (such as an optical tracking system) and an image acquisition device (such as a CT/MRI scanner) in the laboratory environment.

In order to ensure the validity and reliability of the data in the embodiments of the present disclosure, the following requirements and principles need to be met.

### 5-1. Data authenticity.

Herein, data authenticity may include physical consistency and statistical consistency. The physical consistency refers to: generated data should conform to the laws of human anatomy and physiology. For example, the body surface motions and internal organ motions caused by respiration should have a reasonable time delay and amplitude relationship, and the changes in tumor location should be consistent with the respiratory cycle. The statistical consistency refers to: generated data distribution should be consistent with the statistical distribution of real data. For example, the range of motion and frequency distribution of marker points should be consistent with the real patient, and the shape and position distribution of tumors in image data should be consistent with real cases.

### 5-2. Data generated from data diversity should cover as many scenarios and individual differences as possible.

Herein, the data may include: different patient body types, respiratory patterns, tumor position and size; different arrangements of marker points or body contour shapes; different image acquisition conditions (such as resolution, noise level).

In one example, the data diversity may be achieved through the following manners: adjusting parameters (such as a random seed of GAN) of a generation model, introducing random noise or disturbance.

### 5-3. Accuracy of data annotation.

Herein, the accuracy of data annotation refers to that data must have accurate standards, especially a true position of a tumor. The data annotation manner may include: in a physical model, directly recording a true value of the generated data; in deep learning data generation, using post-processing (such as expert validation) to ensure accurate labeling.

### 5-4. Data quality verification.

Herein, the above data needs to undergo strict quality verification to ensure that it may be effectively used for model training. A verification manner may include: comparing with real data, evaluating the authenticity of generated data (such as distribution similarity), training models using generated data, and testing their performance on real data.

### 5-5. Adhere to ethical and privacy standards.

Herein, if the data is based on real patient data, it should be ensured that the data is anonymous to avoid leaking patient privacy. The data generation process should comply with ethical requirements and avoid the misuse of patient data.

In some embodiments, in the embodiments of the present disclosure, the proximal device may further indirectly infer changes in the target volume based on real-time monitoring for a surface motion of the target object. Specifically, it can include the following steps.

In step 1, positions of the target volume and body surface optical data of the target object at different respiratory phases are acquired.

In one example, before performing irradiation on the target volume, the positions and body surface optical data of the target object at different respiratory phases may be acquired through 4D-CT or 4D-MRI scanning.

In step 2, a motion model of the target volume is established based on the positions and body surface optical data of the target object at different respiratory phases.

Herein, the motion model may be a correlation model between the individualized body surface optical data of the target object and the position changes of the target volume. For example, the motion model may be a regression analysis based model or a machine learning based model.

In one example, linear regression, polynomial regression, or other statistical methods may be used to fit the relationship between body surface motion (such as chest and abdominal marker point positions) and changes in the position of the target volume. And based on this relationship, a motion model is constructed.

In another example, machine learning algorithms such as neural networks and support vector machines may be used to train a correlation model between body surface motion and position changes of the target volume.

In the embodiments of the present disclosure, artificial intelligence technology may also be utilized to optimize the correlation model between body surface optical data and the position changes of the target volume. For example, artificial intelligence technology may include deep learning, transfer learning, or the like.

Herein, deep learning may include convolutional neural networks, long short-term memory networks, or the like. Transfer learning refers to the use of transfer learning techniques to quickly adapt to a patient's new data based on their existing data.

In some embodiments, when the irradiation is about to be performed on the target volume, the remote device may use an X-ray projection image to acquire the current position of the target volume and predict the position of the target volume based on the body surface motion model. If there is an offset between the current position of the target volume and a predicted position, the parameters of the second target volume positioning model may be optimized.

Furthermore, in the embodiments of the present disclosure, the target volume positioning system may further include devices such as a respiratory band and a pressure sensor, etc., which may be configured to acquire the patient's respiratory signals and other physiological parameters (such as a heart rate). The target volume positioning model may determine an actual position of the target volume based on the patient's respiratory signals, physiological parameters, and surface motion data.

In one implementation, the target volume positioning system may use respiratory signals as input variables and combine them with body surface motion data to construct a multi-modal motion prediction model. Alternatively, time sequence analysis manners (such as LSTM neural networks) may be used to construct the dynamic relationship between respiratory signals and changes in the position of the target volume.

In the embodiments of the present disclosure, by performing surface optical monitoring on the target object and predicting the position of the target volume, the second target volume positioning model may be dynamically adjusted to facilitate accurate adjustment of the irradiation direction and dose distribution in the future, ensuring that the target volume is always within the optimal therapeutic target volume.

In some other embodiments, in the method provided in the embodiments of the present disclosure, the second target volume positioning model may also be configured to: obtain a dynamic position of the target volume based on the body surface optical data and image data of the target object.

In one example, as shown in FIG. 9, the time sequence of surface optical data and image data features of the target object may be input into the second target volume positioning model to obtain the dynamic position of the target volume.

Herein, the time sequence of surface optical data may include: three-dimensional coordinates of the marker points (such as [x, y, z]) or a point cloud of the body surface contour. The image data may include static imaging data (such as a CT image, an MRI image) and dynamic imaging data (such as 4D-CT data), anatomical information (such as a position, shape) of a tumor, or functional information (such as metabolic activity).

A data format of the time sequence of body surface optical data may be (T, N, S). T represents a time step (frame rate), and N represents a number of body surface marker points or point clouds. S represents three-dimensional coordinates. The data format for the features of image data is usually three-dimensional voxel or two-dimensional slice. For example, the data format may be (C, H, W, D). C represents a number of channels in the image, while H, W, and D represent a height, a width, and a depth of the image, respectively. A format of the dynamic position of the target volume is (T, S1). S1 represents a position of the target volume at each time step.

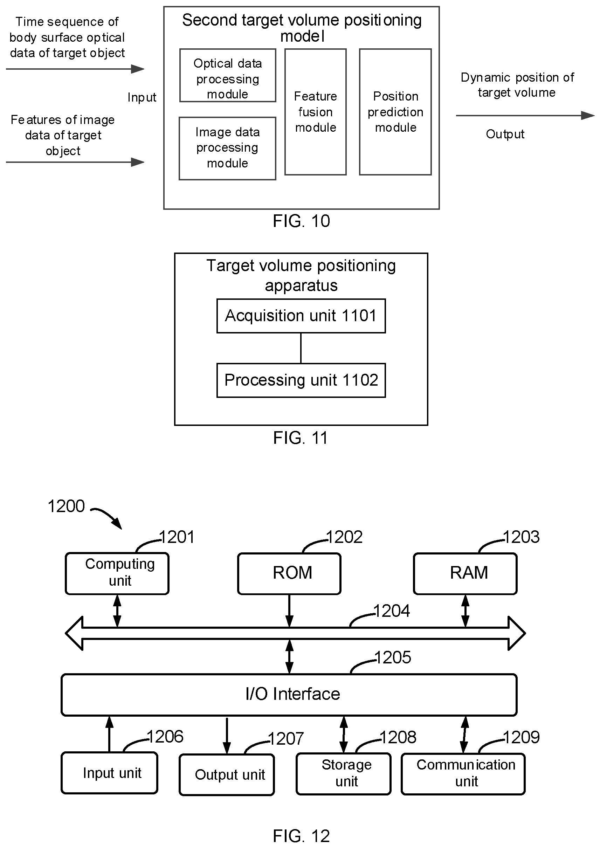

In one implementation, as shown in FIG. 10 and combined with FIG. 9, the second target volume positioning model may include a plurality of modules, the plurality of modules may include an optical data processing module, an image data processing module, a feature fusion module, and a position prediction module.

Herein, the optical data processing module may be configured to: use recurrent neural networks (RNN), long short-term memory networks (LSTM), or gated recurrent units (GRU) to process optical time sequence data. The output of the optical data processing module is: motion features of time sequence, where a format of the output data is (TF), where F is the feature dimension.

The image data processing module may be configured to: use convolutional neural networks (CNN) or 3D-CNN to extract image features. Pre-trained models such as ResNet and UNet may also be used to extract features from images. The output of the image data processing module is: image features. A format of the output data is (F_I), where F_I is a dimension of an image feature.

The feature fusion module may be configured to fuse motion features and image features of the body surface optical data. For example, the motion features and the image features may be directly concatenated into one vector. For example, weights of optical features and image features may be dynamically adjusted through attention mechanisms (such as Transformer). For example, deep cross networks (DCNs) or other interaction mechanisms may be used to model the nonlinear relationship between two kinds of features.

The position prediction module may be configured to output the dynamic position of the tumor using fully connected layers (MLP) or regression layers. A format of the output data is: (T, 3), T represents a tumor position ([x, y, z]) at each time step.

The solutions in the embodiments of the present disclosure are introduced above from the perspective of methods. It could be understood that, in order to implement the above functions, the proximal device includes corresponding hardware structures and/or software modules for implementing various functions. Those skilled in the art should easily realize that the embodiments of the present disclosure are able to be implemented in the form of hardware or a combination of hardware and computer software in combination with units and steps of algorithms described in the embodiments disclosed in the present disclosure. Whether a certain functionality is implemented by hardware or by computer software driving hardware depends on the specific application and design constraint conditions of the technical solutions. A professional technician may use different methods to implement the described functions for each specific application, but such implementation should not be considered beyond the scope of the embodiments of the present disclosure.

The embodiments of the present disclosure may divide the proximal device into functional units according to the above method examples. For example, division of various functional units may be performed corresponding to various functions, or two or more functions may be integrated into one processing unit. The above integrated unit may be implemented in the form of hardware, or may be implemented in the form of a software functional unit. It should be noted that the division of the units in the embodiments of the present disclosure is illustrated, which is only a logical functional division, and there may be other division manners in an actual implementation.

As shown in FIG. 11, a target volume positioning apparatus is provided in the embodiments of the present disclosure, and the target volume positioning apparatus may be applied to a proximal device or may be a proximal device. The target volume positioning apparatus includes: an acquisition unit 1101 and a processing unit 1102.

The acquisition unit 1101 is configured to acquire current image data of a target object before irradiation is to be performed on a target volume of the target object. The processing unit 1102 is configured to input the current image data into a first target volume positioning model, where the first target volume positioning model is configured to determine a position offset of the target volume based on a dynamic mapping relationship for the target volume; the dynamic mapping relationship for the target volume is: a correspondence between a position of the target volume of the target object and the image data with motion changes of the target volume.

In some embodiments, the current image data includes: body surface optical data and/or an X-ray projection image.

In some embodiments, the acquisition unit 1101 is further configured to: acquire the dynamic mapping relationship for the target volume; where the dynamic mapping relationship for the target volume is determined by a second target volume positioning model based on a plurality of kinds of modalities of medical image data for the target object acquired during a positioning stage.

In some embodiments, the second target volume positioning model is further configured to: before performing the irradiation on the target volume of the target object, determine the target volume according to the plurality of kinds of modalities of medical image data for the target object; and generate an irradiation plan for the target volume according to the target volume.

In some embodiments, the plurality of kinds of modalities of medical image data include body surface optical data and at least one of the following: an X-ray projection image, a CT image, an MRI image, a PET image, a PET-CT image, a PET-MRI image, a SPECT image, a SPECT-CT image, a US image, a DSA image, a pathological slice image.

In some embodiments, the first target volume positioning model is obtained by performing knowledge distillation for the second target volume positioning model.

In some embodiments, the knowledge distillation includes one or more of the following: medical image data distillation; model parameter distillation; or irradiation strategy distillation.

In some embodiments, the processing unit 1102 is further configured to: after the irradiation on the target volume is completed, feed irradiation data back to the second target volume positioning model, so that the second target volume positioning model is updated based on the irradiation data.

In some embodiments, the irradiation data includes at least one of the following: abnormal information during a process of the irradiation; adjustment information for an irradiation strategy during the process of the irradiation; or a correspondence between a position offset and an irradiation dose for the target volume during the process of the irradiation.

In some embodiments, the processing unit 1102 is further configured to: update the first target volume positioning model and the second target volume positioning model based on federated learning.

In some embodiments, the first target volume positioning model is further configured to: in response to that the position offset of the target volume is within a range of a preset threshold, output first indication information for indicating to perform the irradiation on the target volume.

In some embodiments, the first target volume positioning model is further configured to: in response to that the position offset of the target volume is not within the range of the preset threshold, output second indication information for adjusting the position of the target volume.

In some embodiments, the processing unit 1102 is further configured to: in response to that a time interval between an acquisition time for the current image data and an outputting time for the first indication information is shorter than a preset time interval, control a radiation therapy device to perform the irradiation on the target volume.

In some embodiments, the acquisition unit 1101 is further configured to: acquire a radiation therapy guidance image of the target object during a process of acquiring the current image data of the target volume; and the processing unit 1102 is further configured to: input the current image data and the radiation therapy guidance image into the first target volume positioning model, to determine the position offset of the target volume.

In some embodiments, the processing unit 1102 is further configured to: determine a state of the target object based on the current image data of the target volume, where the state of the target object includes an abnormal motion state and/or a breath holding state; above-mentioned inputting the current image data into the first target volume positioning model includes: in response to that the state of the target object meets a preset state, inputting the current image data into the first target volume positioning model.

In some embodiments, the processing unit 1102 is further configured to: in response to the target object is in a breath holding state without an abnormal motion state, perform the irradiation on the target volume.

In some embodiments, the target volume is a tumor biological target volume.

In some embodiments, the method in the present disclosure is applied to each fractionated radiation therapy in a plurality of fractionated radiation therapies.

According to the embodiments of the present disclosure, an electronic device is further provided in the present disclosure, which includes at least one processor; and a memory connected with the at least one processor in communication; where the memory stores instructions that are capable of being executed by the at least one processor, and the instructions are executed by the at least one processor to enable the at least one processor to perform the target volume positioning method provided in the present disclosure.

According to the embodiments of the present disclosure, a non-transitory computer-readable storage medium storing computer instructions are further provided in the present disclosure, where the computer instructions are used to cause an electronic device to perform the target volume positioning method provided in the present disclosure.

According to the embodiments of the present disclosure, a computer program product is further provided in the present disclosure, which includes computer programs that, when executed by a processor, implement the target volume positioning method provided in the present disclosure.

FIG. 12 shows a schematic block diagram of an example electronic device 1200 that may be configured to implement the embodiments of the present disclosure. An electronic device is intended to represent various forms of digital computers, such as laptops, desktops, workstations, personal digital assistants, servers, blade servers, mainframes, and other appropriate computers. The electronic device may also represent various forms of mobile devices, such as cellular phones, smart phones, wearable devices, and other similar computing devices. The components shown herein, their connections and relationships, and their functions, are intended only as examples, and are not meant to limit implementations of the present disclosure described and/or claimed herein. In some embodiments, the electronic device may be the proximal device shown in above-mentioned FIG. 1.

As shown in FIG. 12, the electronic device 1200 includes a computing unit 1201, which may perform various appropriate actions and processes according to computer programs stored in a read-only memory (ROM) 1202 or computer programs loaded from a storage unit 1208 to a random access memory (RAM) 1203. In RAM 1203, various programs and data required for operations of the electronic device 1200 may also be stored. The computing unit 1201, ROM 1202, and RAM 1203 are connected to each other through the bus 1204. An input/output (I/O) interface 1205 is also connected to the bus 1204.

Multiple components in the electronic device 1200 are connected to the input/output interface 1205, and include: an input unit 1206 (such as a keyboard, a mouse, etc.); an output unit 1207 (such as various types of displays, speakers, etc.); a storage unit 1208 (such as a disk, an optical disk, etc.); and a communication unit 1209 (such as a network card, a modem, a wireless communication transceiver, etc.). The communication unit 1209 allows the electronic device 1200 to exchange information/data with other devices through computer networks such as the Internet and/or various telecommunications networks.

The computing unit 1201 may be a various general and/or special processing components having processing and computing capabilities. Some examples of the computing unit 1201 include, but are not limited to, a central processing unit, a graphics processing unit (GPU), various dedicated artificial intelligence (AI) computing chips, various computing units that run machine learning model algorithms, a digital signal processor, and any appropriate processors, controllers, microcontrollers, etc. The computing unit 1201 executes the various methods and processes as described above, such as the target volume positioning method. For example, in an embodiment, the target volume positioning method may be implemented as a computer software program, which is tangibly included in a machine-readable medium, such as storage unit 1208. In an embodiment, a portion or all of the computer programs may be loaded and/or installed on the electronic device 1200 via the ROM 1202 and/or the communication unit 1209. When the computer programs are loaded into the RAM 1203 and executed by the computing unit 1201, one or more steps of the target volume positioning method described above may be performed. Alternatively, in other embodiments, the computing unit 1201 may be configured to perform the target volume positioning method in any other appropriate manners (e.g., by means of firmware).

Various implementations of the systems and techniques described above in the present document may be realized in a digital electronic circuit system, an integrated circuit system, a field programmable gate array, an application specific integrated circuit, application specific standard parts (ASSP), a system on chip (SOC), a complex programmable logic device (CPLD), a computer hardware, firmware, software, and/or combinations thereof. These various implementations may include: being implemented in one or more computer programs, where the one or more computer programs may be executed and/or interpreted on a programmable system including at least one programmable processor, and a programmable processor may be a special purpose or general purpose programmable processor that may receive data and instructions from a storage system, at least one input device, and at least one output device, and transmit data and instructions to the storage system, the at least one input device, and the at least one output device.

Program codes for implementing the methods of the present disclosure may be written in any combination of one or more programming languages. These program codes may be provided to a processor or a controller of a general-purpose computer, a special-purpose computer or other programmable data processing devices, so that when the program codes are executed by the processor or controller, the functions/operations specified in the flowcharts and/or block diagrams are implemented. The program codes may be executed entirely on the machine, executed partly on the machine, may be as a stand-alone software package, and partly executed on the machine and partly executed on a remote machine, or entirely executed on the remote machine or server.

In the context of the present disclosure, a machine-readable medium may be a tangible medium, which may contain or store programs for use by an instruction execution system, apparatus, or device, or for use in connection with the instruction execution system, apparatus, or device. The machine-readable medium may be a machine-readable signal medium or a machine-readable storage medium. A machine-readable medium may include, but is not limited to, an electronic, magnetic, optical, electromagnetic, infrared, or semiconductor system, apparatus, or device, or any suitable combination thereof. More specific examples of the machine-readable storage medium may include electrical connections based on one or more wires, a portable computer disk, a hard disk, a random access memory, a read-only memory, an erasable programmable read-only memory, optical fibers, a portable compact disk read-only memory, an optical storage device, a magnetic storage device, or any suitable combination thereof.

To provide interaction with a user, systems and techniques described herein may be implemented on a computer, and the computer has: a display apparatus (such as a cathode ray tube (CRT) or a liquid crystal display (LCD) monitor) for displaying information to the user; and a keyboard and a pointing apparatus (such as a mouse or trackball), and the user may provide input to the computer through the keyboard and the pointing apparatus. Other types of apparatuses may also be configured to provide interaction with the user; for example, a feedback provided to the user may be any form of sensory feedback (e.g., a visual feedback, an auditory feedback, or a tactile feedback); and input from the user may be received in any form (including an acoustic input, a voice input, or a tactile input).

The systems and techniques described herein may be implemented in a computing system that includes back-end components (e.g., as a data server), or a computing system that includes middleware components (e.g., an application server), or a computing system that includes front-end components (e.g., a user computer with a graphical user interface or a web browser, and a user may interact with implementations of the systems and techniques described herein through the web browser), or a computing system that includes any combination of such back-end components, middleware components, or front-end components. The components of the system may be interconnected by any form or media of digital data communication (e.g., a communication network). Examples of the communication network include a local area network (LAN), a wide area network (WAN), and the Internet.

A computer system may include a client and a server. The client and server are generally remote from each other and typically interact with each other through the communication network. A relationship of the client and server is arisen by computer programs running on the corresponding computers and having a client-server relationship to each other. The server may be a cloud server, or also may be a server of a distributed system, or a server combined with blockchains.

It should be understood that various forms of the processes shown above may be used with steps being reordered, added or deleted. For example, the various steps described in the present disclosure may be executed in parallel, or may also be executed sequentially, or may be executed in different orders, as long as the expected results of the technical solutions in the present disclosure may be achieved, and the orders for executing the steps are not limited herein.

The specific implementations mentioned above do not limit the scope of the present disclosure. It should be understood by those skilled in the art that various modifications, combinations, sub-combinations and substitutions may be made according to design requirements and other factors. Any modifications, equivalent substitutions, and improvements made within principles of the present disclosure shall be included within the scope of protection of the present disclosure.

## Claims

1. A target volume positioning method, **characterized by** comprising:
acquiring (S601) current image data of a target object before irradiation is to be performed on a target volume of the target object; and
inputting (S602) the current image data into a first target volume positioning model, wherein the first target volume positioning model is configured to determine a position offset of the target volume based on a dynamic mapping relationship for the target volume; wherein the dynamic mapping relationship for the target volume is: a correspondence between a position of the target volume of the target object and the image data with motion changes of the target volume.

2. The method according to claim 1, wherein the current image data includes: body surface optical data and/or an X-ray projection image.

3. The method according to claim 1, wherein the method further comprises:
acquiring the dynamic mapping relationship for the target volume; wherein the dynamic mapping relationship for the target volume is determined by a second target volume positioning model based on a plurality of kinds of modalities of medical image data for the target object acquired during a positioning stage.

4. The method according to claim 3, wherein the second target volume positioning model is further configured to:
before performing the irradiation on the target volume of the target object, determine the target volume according to the plurality of kinds of modalities of medical image data for the target object; and
generate an irradiation plan for the target volume according to the target volume.

5. The method according to claim 3 or 4, wherein the plurality of kinds of modalities of medical image data include body surface optical data and at least one of the following:
an X-ray projection image, a computed tomography (CT) image, a magnetic resonance imaging (MRI) image, a positron emission tomography (PET) image, a PET-CT image, a PET-MRI image, a single photon emission computed tomography (SPECT) image, a SPECT-CT image, an ultrasound imaging (US) image, a digital subtraction angiograph (DSA) image, a pathological slice image.

6. The method according to claim 3, wherein the first target volume positioning model is obtained by performing knowledge distillation for the second target volume positioning model, wherein the knowledge distillation includes one or more of the following:
medical image data distillation;
model parameter distillation; or
irradiation strategy distillation.

7. The method according to claim 3, wherein the method further comprises:
after the irradiation on the target volume is completed, feeding irradiation data back to the second target volume positioning model, so that the second target volume positioning model is updated based on the irradiation data, wherein the irradiation data includes at least one of the following:
abnormal information during a process of the irradiation;
adjustment information for an irradiation strategy during the process of the irradiation; or
a correspondence between a position offset and an irradiation dose for the target volume during the process of the irradiation.

8. The method according to claim 3, wherein the method further comprises:
updating the first target volume positioning model and the second target volume positioning model based on federated learning.

9. The method according to claim 1, wherein the first target volume positioning model is further configured to:
in response to that the position offset of the target volume is within a range of a preset threshold, output first indication information for indicating to perform the irradiation on the target volume; or
in response to that the position offset of the target volume is not within the range of the preset threshold, output second indication information for indicating to adjust the position of the target volume; wherein
the method further comprises:
in response to that a time interval between an acquisition time for the current image data and an outputting time for the first indication information is shorter than a preset time interval, controlling a radiation therapy device to perform the irradiation on the target volume.

10. The method according to claim 1, wherein the method further comprises:
acquiring a radiation therapy guidance image of the target object during a process of acquiring the current image data; and
inputting the current image data and the radiation therapy guidance image into the first target volume positioning model, to determine the position offset of the target volume.

11. The method according to claim 1, wherein the method further comprises:
determining (S701) a state of the target object based on the current image data, wherein the state of the target object includes an abnormal motion state and/or a breath holding state;
inputting the current image data into the first target volume positioning model comprises:
in response to that the state of the target object meets a preset state, inputting (S702) the current image data into the first target volume positioning model.

12. The method according to claim 1, wherein the method further comprises:
in response to the target object is in a breath holding state without an abnormal motion state, performing the irradiation on the target volume.

13. The method according to any one of claims 1-4, wherein
the target volume is a tumor biological target volume; or
the target volume positioning method is applied to each fractionated radiation therapy in a plurality of fractionated radiation therapies.

14. A target volume positioning system, **characterized by** comprising: a radiation therapy device, an image acquisition device, and a proximal device;
wherein the radiation therapy device is configured to perform irradiation on a target volume; the image acquisition device is configured to acquire image data of a target object; and the proximal device is configured with a first target volume positioning model, and is configured to perform the method according to any one of claims 1-13.

15. The system according to claim 14, wherein the target volume positioning system further comprises at least one of:
a remote device, wherein the remote device is configured with a second target volume positioning model; or
a positioning device, wherein the positioning device is configured to perform simulated positioning on the target object before the irradiation is about to be performed on the target volume of the target object.
